# EUROPEAN PATENT SPECIFICATION

(11) **EP 2 271 332 B1**
(45) Date of publication and mention of the grant of the patent: **20.06.2012**
(21) Application number: 09727195.1
(22) Date of filing: 01.04.2009
(51) Int. Cl.: A61K 31/198, A61P 25/16, A61P 25/28

(54) **USE OF COMPOUNDS IN THE TREATMENT OF TAUOPATHY**
VERWENDUNG VON VERBINDUNGEN BEI DER BEHANDLUNG VON TAUOPATHY
UTILISATION DE COMPOSÉS POUR LE TRAITEMENT DE TAUOPATHIE

(30) Priority: 01.04.2008 GB 0805862
(43) Date of publication of application: 12.01.2011
(73) Proprietor: Bioalvo - Serviços, Investigação E Desenvolvimento Em Biotecnologia S.A., 1749-016 Lisboa (PT)
(72) Inventor: DOS SANTOS, Alexandre Maria, Barros, P-1749-016 Lisboa (PT); RODRIGUES, Cátia, Santana, Reverendo, P-1749-016 Lisboa (PT); ROCA, Christophe François Aimé, P-1749-016 Lisboa (PT); MOREIRA DE OLIVEIRA VIEIRA, Helena Margarida, P-1749-016 Lisboa (PT); BERNARDO DE SOUSA, José Manuel, P-1749-016 Lisboa (PT); CEREJO, Marta Isabel Heitor, P-1749-016 Lisboa (PT); MENDES DA SILVA CALADO, Patrícia, Ramalhete, P-1749-016 Lisboa (PT); PINHEIRO, Ricardo Filipe, Antunes, P-1749-016 Lisboa (PT); CHATERJEE, Sukalyan, P-1749-016 Lisboa (PT); RIBEIRO, Marta Maria, Batista, P-1649-028 Lisboa (PT); CASTANHO, Miguel Augusto, Rico, Botas, P-1649-028 Lisboa (PT); RODRIGUEZ, Eduard, Bardají, E-17071 Girona (ES); COROMINAS, Montserraat Heras, E-17071 Girona (ES); TAVARES, Isaura, Ferreira, P-4200-319 Porto (PT); PINTO, Marta Sofia, Carvalho, Teixeira, P-4200-319 Porto (PT)
(74) Representative: Cornish, Kristina Victoria Joy
(86) International application number: PCT/PT2009/000018
(87) International publication number: WO 2009/123486

(56) References cited:
- WO-A-98/09985
- WO-A-03/066859
- WO-A-2006/074114
- US-A1- 2006 142 241
- VINCENT A M ET AL: "Identification of candidate drugs for the treatment of ALS" AMYOTHROPHIC LATERAL SCLEROSIS AND OTHER MOTOR NEURON DISORDERS, MARTIN DUNITZ, LONDON, GB, vol. 6, no. 1, 1 January 2005 (2005-01-01), pages 29-36, XP009096168 ISSN: 1466-0822
- BEAN A J ET AL: "[d-Arg]Kyotorphin induced ipsilateral rotation: evidence for in vivo effects independent of Met-enkephalin release" BRAIN RESEARCH, ELSEVIER, AMSTERDAM, NL, vol. 321, no. 2, 12 November 1984 (1984-11-12), pages 327-331, XP024272963 ISSN: 0006-8993 [retrieved on 1984-11-12]

## Description

The present invention relates to derivatives of L-Tyrosyl-L-Arginine and the use of said derivatives in the prevention and/or treatment of a tau-induced cytotoxicity.

The tauopathies are a group of diverse dementias and movement disorders which have as a common pathological feature the presence of intracellular accumulations of abnormal filaments of the tau protein, termed neuroflibrillary tangles (NFT). This group of diseases includes Corticobasal Degeneration (CBD), Frontotemporal Dementia with Parkinsonism linked to Chromosome 17 (FTDP 17), Pick's Disease (PiD), Progressive Supranuclear Palsy (PSP) and Alzheimer's Disease (AD). Additionally, accumulation of tau into inclusions has been reported in Multiple System Atrophy (MSA), Parkinson's Disease (PD), Dementia with Lewy Bodies (DLB), Down's Syndrome and Parkinsonism-Dementia Complex of Guam. The normal tau protein controls the orientation and stability of microtubules in neurons, astrocytes and oligodendrocytes. Microtubules are involved with other components of the cytoskeleton in basic cellular processes such as aggregation of genetic material, maintenance of cell shape, intracellular transport, etc. The NFTs observed in tauopathies are enriched in abnormally hyperphosphorylated tau, which is less competent in binding to and stabilizing microtubules. Under normal physiological conditions, there exists a dynamic equilibrium between the pools of microtubule-bound tau and free (unbound) cytosolic tau. Under pathological conditions this equilibrium is shifted towards an increase in the level of unbound tau, favouring its aberrant folding and aggregation. The disengagement of tau from the microtubules is the triggering event in the aggregation cascade and can be caused by numerous factors, including mutations in tau, increases in the level of tau expression, increases in the rate of tau phosphorylation and decreases in the rate of tau dephosphorylation. As a result, a variety of deleterious processes is triggered, such as a destabilised microtubule network, impaired axonal transport, the formation of NFTs and neuronal cell death.

Kyotorphin (L-Tyrosyl-L-Arginine; KTP) was first discovered in 1979 and reported as an endogenous analgesic agent in the brain. However, attempts to utilise Kyotorphin as an analgesic have been unsuccessful due to the inability of Kyotorphin to cross the Blood-brain-barrier (BBB). Attempts have been made to improve the efficacy of Kyotorphin as an analgesic by modifying Kyotorphin to improve its bioavailability. However, such attempts, including derivatisation of Kyotorphin with hydrophobic groups, have not improved the bioavailability of Kyotorphin.

The present invention provides a derivatised form of Kyotorphin which can be used to treat tau-induced cytotoxicities, presently also called tauopathy

The first aspect of the invention relates to a compound of formula (I) 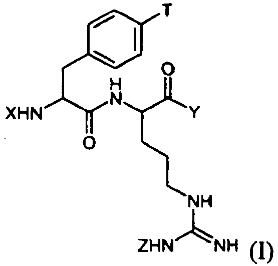
wherein X is hydrogen, R¹, R¹C(O) or R¹CO₂, ,
wherein R¹ is C₁₋₂₀ alkyl, aryl, arylalkyl, alkoxy or arylalkyloxy,
wherein Y is OR², NHR³ or N(R³)₂,
wherein R² is C₁₋₂₀alkyl and each R³ is independently hydrogen or a C₁₋₄ alkyl;
wherein T is OR⁴, NHR⁵ or N(R²)₂,
wherein R⁴ is hydrogen or C₁₋₂₀ alkyl and each R⁵ is independently hydrogen or a C₁₋₄ alkyl;
wherein Z is hydrogen, R⁶, R⁶C(O) or R⁶CO₂,
wherein R⁶ is C₁₋₂₀ alkyl, aryl, arylalkyl, alkyloxy or arylalkyloxy,
for use in the prevention and/or treatment of a tau-induced cytotoxicity.

The compounds of the present invention are particularly provided for the prevention and/or treatment of tau-induced toxicities including Corticobasal Degeneration (CBD), Frontotemporal Dementia with Parkinsonism linked to Chromosome 17 (FTDP 17), Pick's Disease (PiD), Progressive Supranuclear Palsy (PSP), Alzheimer's Disease (AD), Multiple System Atrophy (MSA), Parkinson's Disease (PD), Dementia with Lewy Bodies (DLB), Down's Syndrome, Parkinsonism-Dementia Complex of Guam or any other pathology caused by alterations in the tau protein.

It will be appreciated that the compound of formula (I) is a derivatised form of kyotorphin (L-Tyrosyl-L-Arginine). Preferably, the invention relates to a compound of formula (I) wherein X is hydrogen, Y is hydroxy or NH₂, Z is hydrogen and T is hydroxyl with the proviso that when X and Z are hydrogen and T is hydroxy, Y is not hydroxy.

In a particular embodiment, the invention relates to the use of a compound of formula (I)
- wherein X and Z are hydrogen, T is hydroxyl and Y is NH₂ (in particular L-Tyr-D-Arg-NH₂, D-Tyr-D-Arg-NH₂, or D-Tyr-L-Arg-NH₂) or
- wherein X is methyl, Z is hydrogen, T is hydroxyl and Y is NH₂ (in particular methyl-L-Tyr-L-Arg-NH₂, methyl-L-Tyr-D-Arg-NH₂, methyl-D-Tyr-L-Arg-NH₂ or methyl-D-Tyr-D-Arg-NH₂).

For the purposes of the present invention, a "C₁₋₂₀ alkyl group" as used herein is an alkyl group that is a straight or branched chain with 1 to 20 carbons. The alkyl group therefore has 1, 2, 3, 4, 5, 6, 7, 8, 9, 10, 11, 12, 13, 14, 15, 16, 17, 18, 19 or 20 carbon atoms. The alkyl group can be optionally saturated at one or more positions along the carbon chain. The alkyl group can be hydroxylated at one or more positions along its length. Preferably, the alkyl group has from 1 to 10 carbon atoms, more specifically from 1 to 6 carbon atoms. Specifically, examples of "C₁₋₆ alkyl group" include methyl group, ethyl group, n-propyl group, iso-propyl group, n-butyl group, iso-butyl group, sec-butyl group, tert-butyl group, n-pentyl group, 1,1-dimethylpropyl group, 1,2-dimethylpropyl group, 2,2-dimethylpropyl group, 1-ethylpropyl group, n-hexyl group, 1-methyl-2-methylpropyl group, 1,1,2-trimethylpropyl group, 1-ethylbutyl group, 1-methylbutyl group, 2-methylbutyl group, 1,1-dimethylbutyl group, 1,2-dimethylbutyl group, 2,2-dimethylbutyl group, 1,3-dimethylbutyl group, 2,3-dimethylbutyl group, 2-ethylbutyl group, 2-methylpentyl group, 3-methylpentyl group and the like. For the purposes of the present invention, a "C₁₋₄ alkyl group" is an alkyl group as defined above with 1, 2, 3 or 4 carbon atoms. Examples of "C₁₋₄ alkyl group" include methyl group, ethyl group, n-propyl group, iso-propyl group, n-butyl group, iso-butyl group, sec-butyl group, and tert-butyl group. The alkyl group can be optionally interrupted by one or more oxygen atoms, preferably 1 to 4 oxygen atoms, more preferably 1 or 2 oxygen atoms.

The aryl group is preferably a "C₆₋₁₀ aryl group", i.e. an aryl group constituted by 6, 7, 8, 9 or 10 carbon atoms. For the purposes of the invention, the aryl group includes condensed ring groups such as monocyclic ring group, or bicyclic ring group and the like. Specifically, examples of "C₆₋₁₀ aryl group" include phenyl group, indenyl group, naphthyl group or azulenyl group and the like. It should be noted that condensed rings such as indan and tetrahydro naphthalene are also included in the aryl group. The aryl group is optionally substituted with 1-4 substituent(s) selected from halogen, an oxo group, an ethylenedioxy group, methyl group, ethyl group, butyl group, methoxy group, methylamino group or dimethylamino group.

The arylalkyl group can be positioned such that the aryl or the alkyl group is the most remote from the molecule.

The alkoxy group is preferably a "C₁₋₆ alkyloxy group" meaning an oxy group that is bonded to an alkyl group (as previously defined). Specifically, examples of "C₁₋₆ alkoxy group" include methoxy group, ethoxy group, n-propoxy group, iso-propoxy group, n-butoxy group, iso-butoxy group, sec-butoxy group, tert-butoxy group, n-pentyloxy group, iso-pentyloxy group, sec-pentyloxy group, n-hexyloxy group, iso-hexyloxy group, 1,1-dimethylpropoxy group, 1,2- 1 dimethylpropoxy group, 2,2-dimethylpropoxy group, 2-methylbutoxy group, 1-ethyl-2-methylpropoxy group, 1,1,2-trimethylpropoxy group, 1,1-dimethylbutoxy group, 1,2-dimethylbutoxy group, 2,2-dimethylbutoxy group, 2,3-dimethylbutoxy group, 1,3-dimethylbutoxy group, 2-ethylbutoxy group, 2-methylpentyloxy group, 3-methylpentyloxy group and the like.

The arylaklyloxy group is an alkyloxy group as defined here, together with an attached aryl group. The arylalkyloxy group can be positioned so that the aryl group or the alkyloxy group is the most remote from the molecule.

It will be appreciated that the compounds of formula (I) are derivatives of the dipeptide Tyrosyl-Arginine. For the purpose of the present invention, the amino acid monomers tyrosine and arginine can independently be in the L or D configuration. The present invention therefore encompasses compounds of formula (I) comprising the backbone L-Tyrosyl-L-Arginine, L-Tyrosyl-D-Arginine, D-Tyrosyl-L-Arginine or D-Tyrosyl-D-Arginine. The compound of formula (I) may comprise L-Tyrosil-L-Arginine.

The second aspect of the invention provides the use the compound of formula (I) in the manufacture of a medicament for the prevention and/or treatment of a tau-induced cytotoxicity.

The third aspect of the invention provides a pharmaceutical composition comprising the compound of formula (I) and a pharmaceutically acceptable excipient for use in the prevention and/or treatment of a tau-induced cytotoxicity.

For the purposes of this invention, the pharmaceutically acceptable excipient may be a pharmaceutically acceptable carrier and/or a pharmaceutically acceptable diluent. Suitable carriers and/or diluents are well known in the art and include pharmaceutical grade starch, mannitol, lactose, magnesium stearate, sodium saccharin, talcum, cellulose, glucose, sucrose (or other sugar), magnesium carbonate, gelatin, oil, alcohol, detergents, emulsifiers or water (preferably sterile). The composition may be a mixed preparation of a composition or may be a combined preparation for simultaneous, separate or sequential use (including administration).

For formulation, a diluent, a binder, a disintegration agent, a lubricant, a colorant and a flavoring agent used in general, and as necessary, additives such as a stabilizer, an emulsifier, an absorption enhancer, a surfactant, a pH adjuster, an antiseptic agent, and an antioxidant can be used. In addition, formulation is also possible by combining ingredients that are used in general as raw materials of pharmaceutical formulation, by the conventional method. Examples of these ingredients include (1) soybean oil, animal oil such as beef tallow and synthethic glyceride; (2) hydrocarbon such as liquid paraffin, squalane and solid paraffin; (3) an ester oil such as octyldodecylmyristate and isopropylmyristate; (4) higher alcohol such as cetostearylalcohol and behenyl alcohol; (5) a silicon resin; (6) a silicon oil; (7) a surfactant such as polyoxyethylene fatty acid ester, sorbitan fatty acid ester, glycerin fatty acid ester, polyoxyethylene sorbitan fatty acid ester, polyoxyethylene hardened castor oil and polyoxyethylene polyoxypropylene block co-polymer; (8) a water-soluble polymer such as hydroxyethyl cellulose, polyacrylic acid, carboxyvinyl polymer, polyethyleneglycol, polyvinylpyrrolidone and methyl cellulose; (9) lower alcohol such as ethanol and isopropanol; (10) multivalent alcohol such as glycerin, propylene glucol, dipropylene glycol and sorbitol; (11) a sugar such as glucose and cane sugar; (12) an inorganic powder such as anhydrous silicic acid, magnesium aluminium silicate and aluminium silicate; and (13) purified water and the like.

Among the aforementioned additives, use can be made of 1) lactose, corn starch, sucrose, glucose, mannitol, sorbit, crystalline cellulose, silicon dioxide and the like as a diluting agent; 2) polyvinyl alcohol, polyvinyl ether, methyl cellulose, ethyl cellulose, gum arabic, traganth, gelatine, shellac, hydroxypropyl cellulose, hydroxypropylmethyl cellulose, polyvinylpyrrolidone, polypropyleneglycol.polyoxyethylene block co-polymer, meglumine, calcium citrate, dextrin, pectin and the like as a binder; 3) a starch, agar, gelatine powder, crystalline cellulose, calcium carbonate, sodium bicarbonate, calcium citrate, dextrin, pectin, calcium carboxymethylcellulose and the like as a disintegration agent; 4) magnesium stearate, talc, polyethyleneglycol, silica, hardened plant oil and the like as a lubricant; 5) a colorant, as long as addition thereof to a pharmaceutical drug is authorized, as a colorant; 6) a cocoa powder, menthol, fragrance, a peppermint oil, a cinnamon powder as a flavoring agent; and 7) an antioxidants whose addition to a pharmaceutical drug is authorized such as ascorbic acid and α-tocophenol as an antioxidant.

A compound of formula (I) according to the invention for use in the aforementioned indications may be administered by any convenient way, for example by oral (including by inhalation), parenteral, mucosal (e.g. buccal, sublingual, nasal), vaginal, rectal or transdermal administration and the compositions adapted accordingly.

Preferably the compounds of the invention are provided for systemic administration and preferably not provided for topical application or administration. The present invention therefore preferably relates to the provision of compound of formula (I) for enteral or parenteral administration. Enteral routes of administration include oral (including inhalation), mucosal (including buccal, sublingual, nasal), vaginal or rectal. More particularly, the compound of formula (I) can be administered by intravenous administration, intraarterial administration, intramuscular administration, intracardiac administration; subcutaneous administration, intraosseious infusion, intradermal administration, intrathecal administration, intraperitoneal administration, tranmucosal administration, epidural administration and/or by intravitreal administration.

A compound of formula (I) according to the present invention can be provided in a delayed release composition in combination with a delayed release component to allow targeted release of the compound of formula (I) into the lower gastrointestinal tract for example into the small intestine, the large intestine, the colon and/or the rectum. The delayed release component may comprise an enteric or pH dependent coating, hydrophobic or gelling excipients or coatings, by time dependent hydrogel coatings and/or by acrylic acid linked to azoaromatic bonds coatings.

For oral administration, the compound can be formulated as liquids or solids, for example solutions, syrups, suspensions, emulsions, tablets, capsules, lozenges, dry powder and/or granules.

A liquid formulation will generally consist of a suspension or solution of the compound or physiologically acceptable salt in a suitable aqueous or non-aqueous liquid carrier(s) for example water, ethanol, glycerol, polyethylene glycol or an oil. The formulation may also contain a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and microcrystalline cellulose.

A composition in the form of a capsule can be prepared using routine encapsulation procedures. For example, powders, granules or pellets containing the active ingredient can be prepared using standard carriers and then filled into a capsule, for example a hard gelatin capsule, a HPMC capsule, a soft gelatin capsule etc; alternatively, a dispersion or suspension can be prepared using any suitable pharmaceutical carrier(s), for example aqueous gums, celluloses, silicates or oils and the dispersion or suspension then filled into a soft gelatin capsule.

Compositions for oral administration may be designed to protect the active ingredient against degradation as it passes through the alimentary tract, for example by an outer coating of the formulation on a tablet or capsule.

Typical parenteral compositions consist of a solution or suspension of the compound or physiologically acceptable salt in a sterile aqueous carrier or non-aqueous or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

Compositions for nasal or oral administration may conveniently be formulated as aerosols, drops, gels and powders. Aerosol formulations typically comprise a solution or fine suspension of the active substance in a physiologically acceptable aqueous or non-aqueous solvent and are usually presented in single or multidose quantities in sterile form in a sealed container, which can take the form of a cartridge or refill for use with an atomising device. Alternatively the sealed container may be a unitary dispensing device such as a single dose nasal inhaler or an aerosol dispenser fitted with a metering valve which is intended for disposal once the contents of the container have been exhausted. Where the dosage form comprises an aerosol dispenser, it will contain a pharmaceutically acceptable propellant. The aerosol dosage forms can also take the form of a pump-atomiser.

Compositions suitable for buccal or sublingual administration include tablets, lozenges and pastilles, wherein the active ingredient is formulated with a carrier such as sugar and acacia, tragacanth, or gelatin and glycerin.

Compositions for rectal or vaginal administration are conveniently in the form of suppositories (containing a conventional suppository base such as cocoa butter), pessaries, vaginal tabs, foams or enemas.

Compositions suitable for transdermal administration include ointments, gels and patches, and injections, including powder injections.

Conveniently the composition is in unit dose form such as a tablet, capsule or ampoule.

The composition may contain from 0.1% to 99% (w/w) preferably from 0.1-60% (w/w), more preferably 0.2-20% by weight and most preferably 0.25 to 12% (w/w) of the compound of formula (I), depending on the method of administration.

The amount of the compound of formula (I) effective to treat a tau-induced cytotoxicity as set out above depends on the nature and severity of the cytotoxicity being treated and the weight of the patient in need thereof. The compounds of the invention are to be normally be administered in a daily dosage regimen (for an adult patient) of, for example, an oral dose of between 1 mg and 2000 mg, preferably between 30 mg and 1000 mg, e.g. between 10 and 250 mg or an intravenous, subcutaneous, or intramuscular dose of between 0.1 mg and 100 mg, preferably between 0.1 mg and 50 mg, e.g. between 1 and 25 mg of the compounds of the formula (I) or a physiologically acceptable salt thereof calculated as the free base, the compound being administered 1 to 4 times per day. The unit dose is preferably provided in the form of a capsule or a tablet. Suitably the compounds will be administered for a period of continuous therapy, for example for a week or more. It will be appreciated that the dose ranges set out above provided guidance for the administration of the compound of formula (I) to an adult. The amount to be administered to for example, an infant or a baby can be determined by a medicals practioner or person skilled in the art and can be lower or the same as that administered to an adult.

All preferred features of each of the aspects of the invention apply to all other aspects mutatis mutandis.

It is noted here that in this text, unless otherwise specified, KTP-NH₂ is the compound of the present invention where X is hydrogen, Z is hydrogen, T is hydroxyl and Y is NH₂, in the L-L form.

The invention may be put into practice in various ways and a number of specific embodiments will be described by way of example to illustrate the invention with reference to the accompanying drawings, in which:
Figure 1 shows a partition curve for KTP-NH₂ (X and Z are hydrogen, T is OH and Y is NH₂). Vesicles of zwiterionic lipid, POPC (•), and POPC with negative lipid, POPG in different proportions: 80:20■, 50:50◆ and 30:70 * until 100% POPG (▲). The unit of 1/lw on the y axis is the ratio between fluorescence intensities.
Figure 2 shows the peptide distribution for a representative lipidic system - KTP-NH₂ (1) and two comparative derivatives of KTP (2) and (3) for the purposes of comparison. The x-axis indicates the distance to the bilayer center, being the monolayer thickness 20Å and y-axis represents the values of the probability density function. Traces (4) and (5) relate to the location of the quenching agents used in the study.
Figure 3 shows a Stern-Volmer plot for fluorescence quenching of kyotorphins: KTP (-) and KTP-NH₂ (•);
Figure 4 shows *in vivo* behavioural tests (dose response curves for KTP-NH₂ i.p) in rats (Wister, male) for 'Tail flick' and 'Hot plate';
Figure 5 shows results for oral administration of KTP-NH2 for Tail Flick and Hot Plate tests;
Figure 6 shows the effects of naxolone on rat tail-flick response. KTP-NH₂ and KTP were administered at 3mg/100g, naxolone was administered at 0.5mg/100g;
Figure 7 shows the results of a KTP derivative (control compound 1) incubated with DISAGGREGATOR I P301L (target induced). Cells were grown in 200 microlitre minimum medium supplemented with 20g/L galactose, at 30°C, with agitation at 600 rpm;
Figure 8 shows the results of a KTP derivative (control compound 2) incubated with DISAGGREGATOR I P301L (target induced). Cells were grown as described for figure 7;
Figure 9 shows the results of KTP-NH2 incubated with DISAGGREGATOR I P301 L (target induced). Cells were grown as described for figure 7;
Figure 10 shows the results of KTP-NH2 incubated with DISAGGREGATOR I (chemical induced) submitted to 1 microgram per millilitre tunicamycin. Cells were grown as described for figure 7;
Figure 11 shows the results of KTP-NH2 incubated with DISAGGREGATOR I P301L (target induced). Cells were grown as described for figure 7;
Figure 12 shows the results of KTP-NH2 incubated with DISAGGREGATOR I (chemical induced) submitted to 1 microgram per millilitre tunicamycin. Cells were grown as described for figure 7;
Figure 13 shows the results of KTP-NH2 at 10 micromolar and 100 micromolar on the peak of GFP signal of DISAGGREGATOR I P301L and DISAGGREGATOR I submitted to 0 and 1 microgram per millilitre tunicamycin. Each value is the average of two experiments.
Figure 14 shows the enzyme activity (U/L) of AST, ALT and ALP measured in plasma of rats treated with a diary dose of 3,23mg-100g of body mass during 7 days compared with control animals.
Figure 15 shows the total bilirubin quantity (µmol/L) measured in plasma of rats treated with a diary dose of 3,23mg-100g of body mass during 7 days compared with control animals.
Figure 16 shows the antioxidant capacity of water-soluble constituents in plasma of treated rats. The values are quoted in equivalent ascorbic acid (µmol/L).
Figure 17 shows the antioxidant capacity of lipid-soluble constituents in plasma of treated rats. The values are quoted in equivalent TROLOX (6-Hydroxy-2,5,7,8-tetramethylchromane-2-carboxylic acid) (µmol/L);
Figure 18 shows partition curves for derivatives of KTP-NH2 with improved plasma stability. Vesicles of zwiterionic lipid, POPC, and POPC with negative lipid, POPG, in the proportion 50 POPC: 50 POPG.

### EXAMPLES

### Insertion of KTP-NH2 into a lipid bilayer.

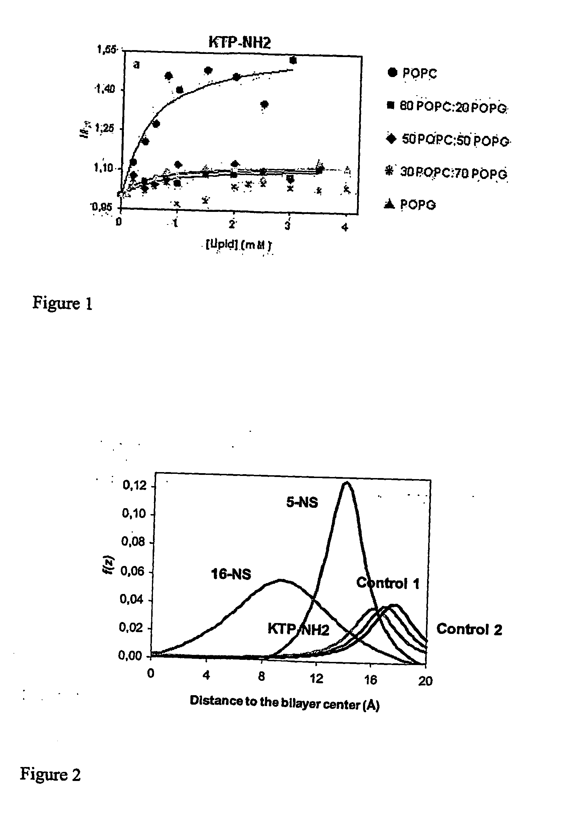

Figure 1 demonstrates the titration of KTP-NH2 with a mammal-mimetic lipid bilayer vesicle (circles). As can be observed, the fluorescence intensity of the phenolic moiety increases due to the insertion in the apolar environment created by the lipids. Negative lipids (other symbols) inhibit the interaction due to charge repulsion. Numerical treatment of the data show that the local concentration in the model membrane of mammals is approx. 2,500 fold higher than in the surrounding bulk aqueous phase, for KTP-NH₂. In fact, the higher partition coefficient for POPC was observed for KTP-NH₂, which is significantly superior to the one of KTP-OH (X and Z are hydrogen, T and Y are OH), for the same membranes (Lopes et al, 2006, J. Phys. Chem. B, 110, 3385-3394).

No evidence was found for aggregation of KTP-NH2 in aqueous medium. KTP-NH2 is therefore suitable for i.v. or i.p. administration. KTP-NH2 diffuses through the blood stream, reaches the BBB, and translocates.

### Depth distribution of the peptides in the membrane

Differential quenching experiments were used to estimate the depth of location of the phenolic ring of the peptides, based on a Brownian dynamics simulation (Fernandes M.X., Garcia de la Torre J., Castanho M.A. 2002. Joint determination by Brownian dynamics and fluorescence quenching of the in-depth location profile of biomolecules in membranes. Anal. Biochem. 307: 1-12.) This methodology allows to determine where the molecules are preferably inserted along the lipid bilayer. The results are shown in Figure 2.The peptides with the amide group show a greater depth of insertion, 4 Å - KTP-NH₂, from the lipid/water interface. This is in agreement with KTP-NH2 having a higher affinity for lipids, as desired.

### Use of acrylamide to determine aggregation of the KTP-NH2 dipeptides

The aggregation of molecules in aqueous medium, as a result of poor solubility, for example, is a critical parameter in pharmacology. As we generated a molecule with increased lipophilicity, KTP-NH2, it was crucial to demonstrate that this molecule and derivatives thereof maintained appropriate solubility characteristics. For this purpose, acrylamide quenching studies were performed (Lakowicz J.R 1999. Principles of Fluorescence spectroscopy, Second Edition ed., Kluwer Academic/Plenum Publishers, New York; Coutinho A and Prieto M. 1993. Ribonuclease T1 and alcohol dehydrogenase fluorescence quenching by acrylamide: A laboratory experiment for undergraduate students J. Chem. Education, 70: 425-428).

Acrylamide is an aqueous quencher of the tyrosine fluorescence. When fluorescent peptides aggregate, the phenolic groups tend to cluster and remain inaccessible to hydrophilic molecules. Conversely, when no aggregation occurs, all tyrosine residues are exposed to the solvent and, therefore, accessible to the contact and quenching by acrilamide.

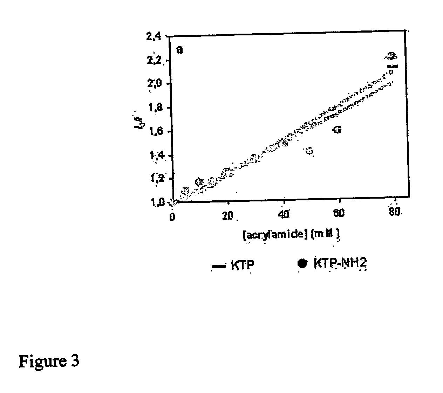

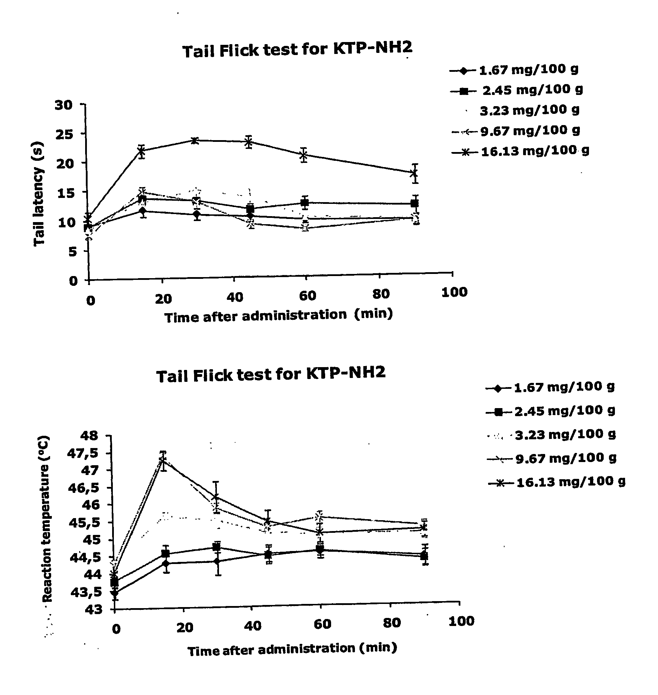

The linearity observed in these plots (as illustrated in figure 3) show that the phenolic groups are always accessible to the hydrophilic molecule acrylamide. The fluorescence spectra were not concentration-dependent (not shown) and there was a linear dependence of fluorescence intensity on concentration (not shown). These results show that no evidence for aggregation of these peptides in aqueous medium was found, indicating favourable solubility properties for KTP-NH2 and tested derivatives.

### Observation of the effect of KTP-NH2 across the Blood Brain Barrier

Kyotorphin is an endogenous analgesic agent in the brain. However, Kyotorphin can not be used as an analgesic due to its inability to cross the blood-brain-barrier. Intra-peritoneal administration of KTP-NH₂ followed by *in vivo* behavioural nociception tests in rats (Wister, male) were used as a demonstration that KTP-NH2 can cross the blood-brain-barrier and therefore have a biological effect in the brain. In addition, KTP-NH2 efficacy after oral administration was also observed.

*In vivo* behavioural anti-nociception tests were carried out in rats (Wister, male). Unless otherwise stated, compounds were administered by intra-peritoneal (i.p.) injection. For most assays, the Tail Flick and the Hot Plate tests were used.

The tail-flick test (D'Amour F.E., Smith D.L. 1941. A method for determining the loss of pain sensation. J Pharmacol Exp Ther 1941;72:74-79) is a standard investigative tool for pain and analgesia assessment in rodents. It is based on a spinal reflex response of the tail to radiant heat. Pain sensitivity in rats was measured as they responded to the application of radiant heat to a small area of their tails. The rat's tail was placed over a window located on a platform and subjected to irradiation by an intense light beam (10 W). When the rat feels discomfort, it flicks its tail which automatically stops the timer. The reaction time from activation of the light beam to the tail flick is automatically presented on a digital display. A cut-off time of 24s was applied. Animal reaction time is a measurement of animal resistance to pain and is used to measure efficacy of analgesics.

The hot-plate test (Eddy N.B and Leimbach D. 1953. Synthetic analgesics II. Dithienylbutenyl and dithienylbutylamines. J. Pharmacol. Exp. Ther. 45: 339) evaluates a supraspinally integrated response in the form of thermal pain reflexes due to footpad contact with a heated surface. During the experiments, the animal is confined in a removable clear acrylic compartment where the latency time to the first hind paw or/and jumping responses are measured. We used a modified hot plate test (Hunskaar S., Berge O.G., Hole K. 1986. A modified hot-plate test sensitive to mild analgesics. Behav. Brain Res. 21: 101-108) in which the temperature was slowly increased (9°C/min) from non-noxious levels (35°C) until a response was observed or a cut-off temperature was reached (52,5°C). The response is the licking of the hindpaw, and the corresponding plate temperature represents the recorded nociceptive end-point. The advantage of Increasing-Temperature Hot-Plate test over the standard constant-temperature hot-plate test is the higher sensitivity and the lack of influences of pre-exposure to the hot-plate before testing. Animal reaction temperature is a measurement of animal resistance to pain and is used to measure efficacy of analgesics.

A KTP derivative was used for comparative purposes, as control. Both drugs were injected i.p. and p.o. and rats were analysed for the Hot Plate and Tail Flick tests.

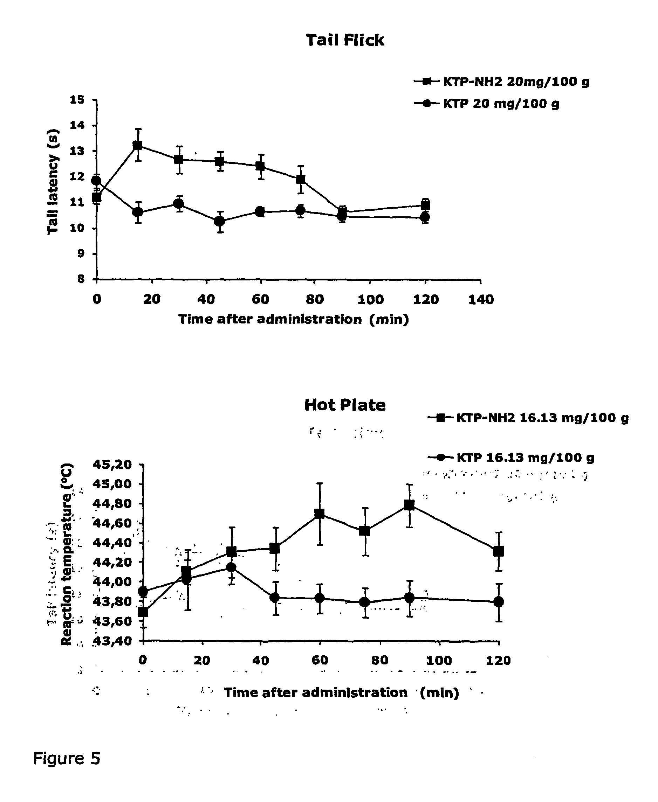

Results are shown in Figure 4 for i.p administration and in Figure 5 for p.o. administration:

KTP-NH2 has a clear anti-nociceptive response, evidenced by the time rats take to remove their tail after stimulus (Tail Flick) or the temperature increase they can freely stand in the paw (Hot Plate). This indicates that KTP-NH2 does cross the blood-brain-barrier and has a biological effect in the brain. Moreover, the observed results validate KTP-NH2 as a compound suitable for both i.p and oral administration.

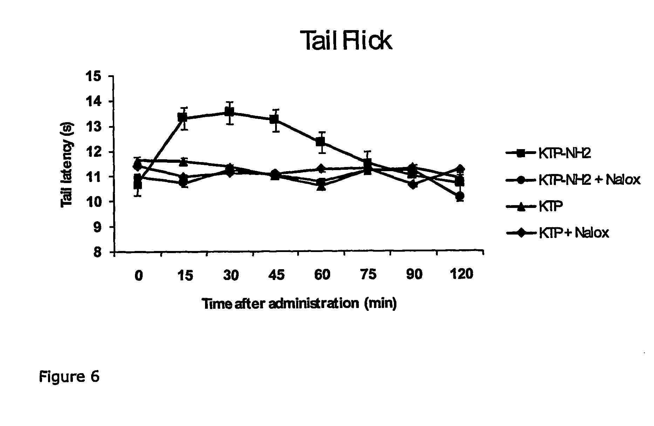

Additionaly, the central action of KTP-NH2 was confirmed by experiments of naloxone-reversible analgesia. Naloxone is a drug that crosses the blood-brain barrier and binds with high affinity to µ-opioid receptors in the central nervous system. Naloxone also has an antagonist action, though with a lower affinity, at κ- and δ-opioid receptors. In order to corroborate the evidence that KTP-NH2 was exerting its analgesic effect *via* a central action, we analyzed the antinociceptive effect of KTP-NH2 after pretreatment with naloxone. Administration of naloxone 10 min before administration of KTP-NH2 completely antagonized the analgesia (Figure 6). These results indicate that KTP-NH2 analgesia is mediated via a central mechanism, i.e. it crosses the blood-brain barrier in order to act in the central nervous system.

### KTP-NH2 as a specific modulator of tau-induced cytotoxicity

KTP-NH2 shows potency against the cellular phenotype induced by the expression of the protein Tau P301L in the yeast platform DISAGGREGATOR I P301L (described in WO/2008/150186 the subject matter of which is incorporated by reference herein). KTP-NH2 was further tested on a general inducer of the same pathological mechanism to assess its specificity for tau and compared with two control KTP derivatives which were tested on the same platform.

The Endoplasmic Reticulum (ER) fulfils multiple cellular functions, the main one being the proper folding of proteins destined for secretion or display on the cell surface. Many disturbances cause accumulation of unfolded proteins in the ER, triggering an evolutionarily conserved response, termed the unfolded protein response (UPR) or ER stress. The initial intent of the UPR is to adapt to the changing environment, and reestablish normal ER function.

The yeast platform DISAGGREGATOR I comprises an ER stress element operably linked to a GFP reporter element and is used to determine the level of ER stress in cells during growth or triggered by specific ER stress inducers, e.g. tunicamycin. Tunicamycin is known to inhibit the glycosylation of nascent polypeptides, resulting in misfolded proteins, that are retained in the endoplasmic reticulum and therefore triggering ER stress.

In parallel, the yeast platform DISAGGREGATOR I P301L comprises the same ER stress element operably linked to the same GFP reporter element and additionally expresses the exogenous protein TAU P301L which induces ER stress.

Using these two platforms in parallel, it is possible to identify compounds that reduce specifically the stress induced by the TAU P301 L protein, and other compounds that are simply general ER stress reducers.

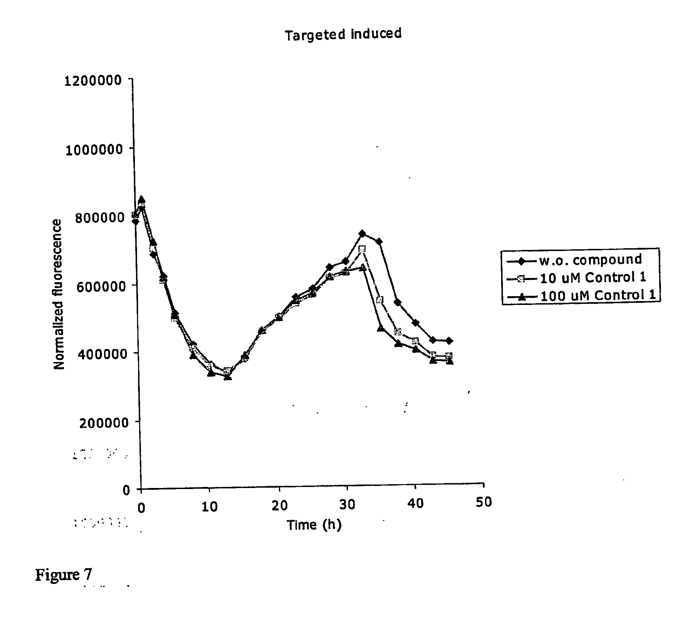

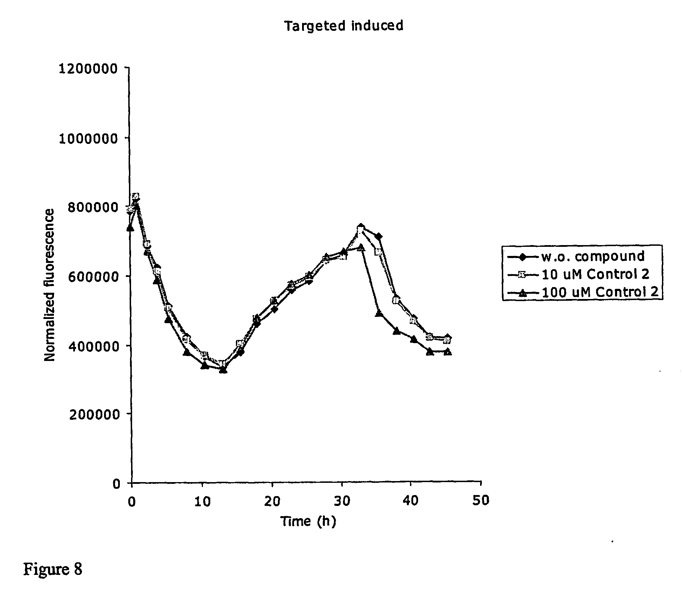

DISAGGREGATOR I P301L cells were grown in 200 µl medium containing KTP-NH2 or control compounds (referred to as control 1 and control 2) at a concentration of 10 µM and 100 µM. No effect on the fluorescence could be detected for the control compounds, meaning that the two compounds are inefficient to reduce the stress triggered by the TAU P301L protein (Figures 7 and 8). However, KTP-NH2 decreased the fluorescence signal.

DISAGGREGATOR I P301L cells were grown in 200 µl medium containing the KTP-NH2 at a concentration of 10 µM and 100 µM. DISAGGREGATOR I cells were grown in the same condition but subjected to an addition of 1 µg/mL of tunicamycin in order to induce ER stress chemically.

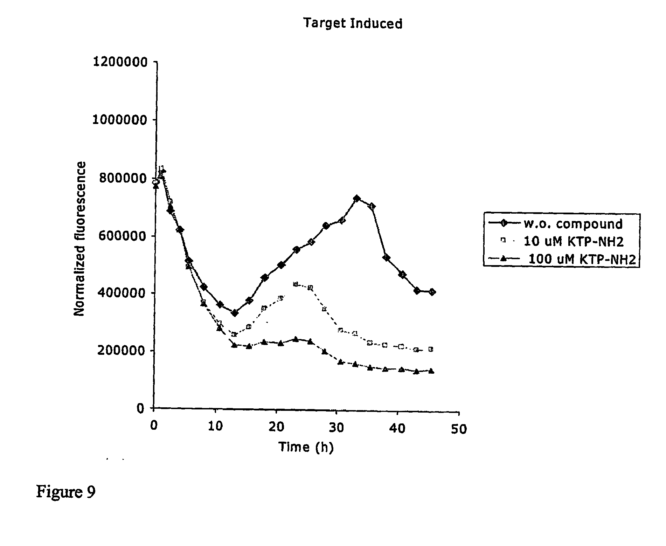

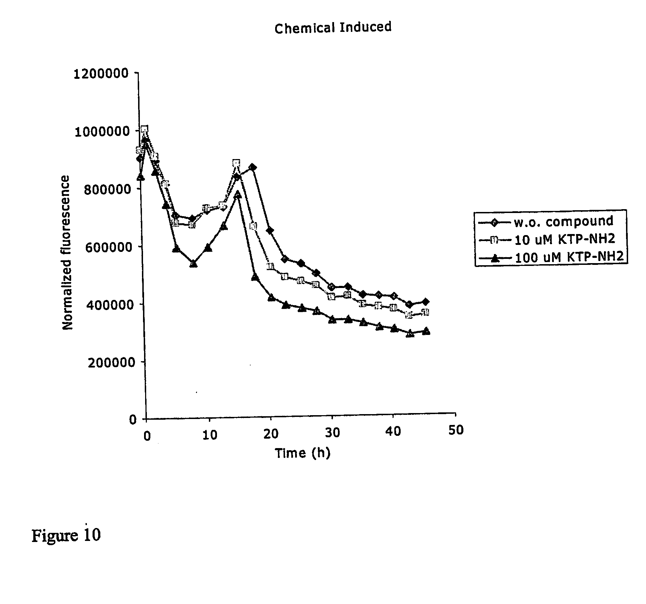

KTP-NH2 decreased the fluorescent signal by 40% at 10 µM and by 65% at 100 µM in DISAGGREGATOR I P301 L cells (Figure 9), indicating a relieve of the ER stress induced by P301L expression. However, KTP-NH2 addition had almost no action on the ER stress chemically induced with 1 µg/ml Tunicamycin in DISAGGREGATOR I cells (Figure 10).

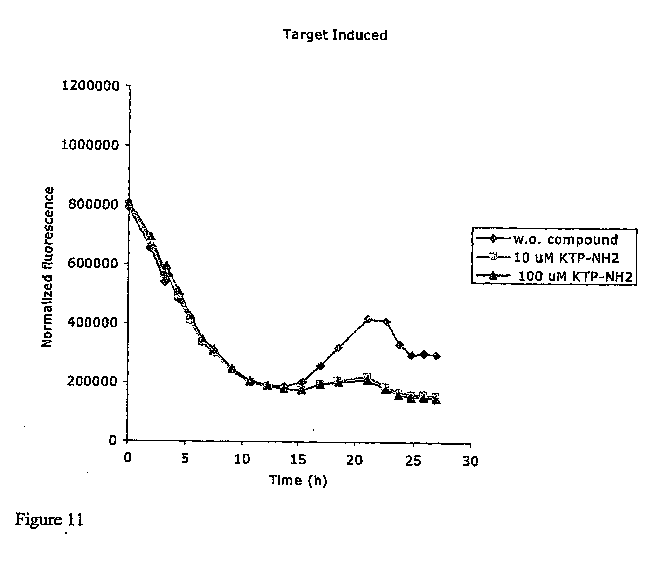

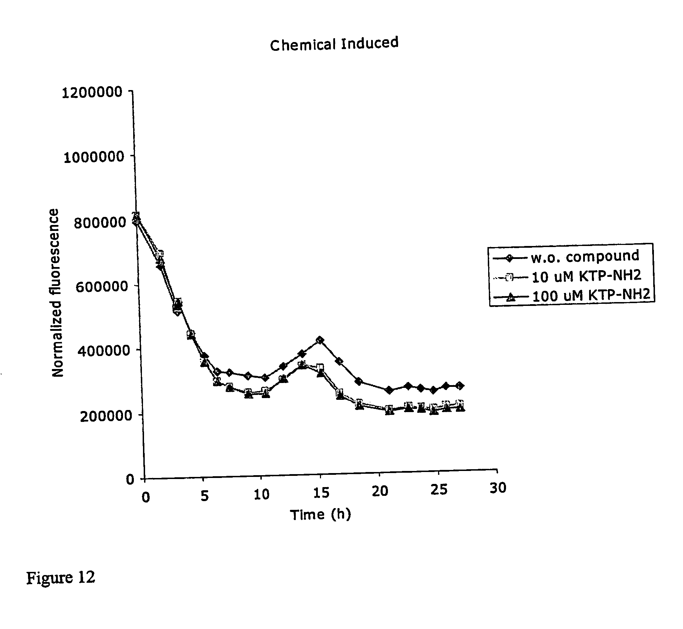

In a second assay, KTP-NH2 decreased the stress by 50% at both 10 µM and at 100 µM in DISAGGREGATOR I P301L cells (Figure 11). In comparison, it did not significantly decrease the stress chemically induced with 1 µg/ml tunicamycin in DISAGGREGATOR I cells (Figure 12).

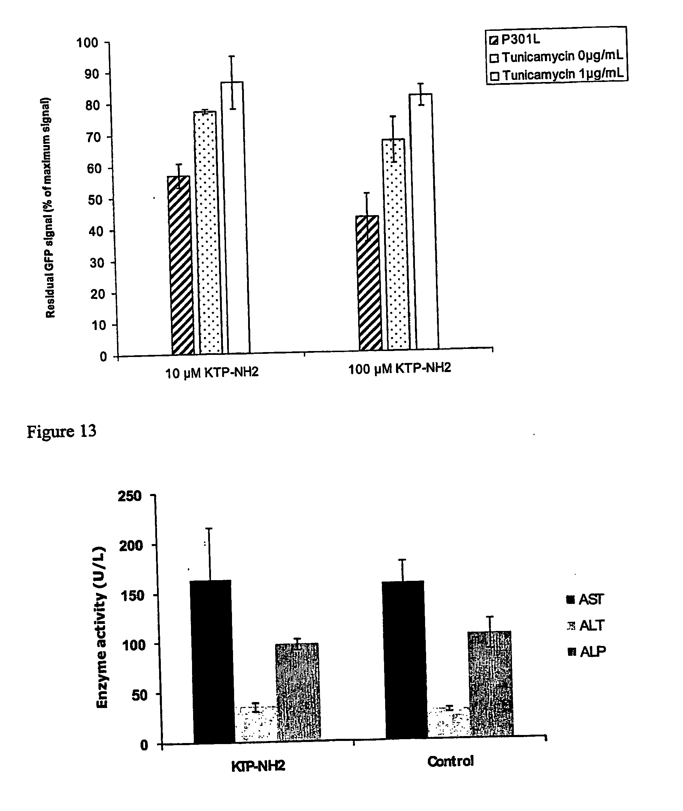

Figure 13 summarises the effect of KTP-NH2 at 10 and 100 µM in all the conditions tested and represents the residual ER stress signal, compared to the maximum signal obtained without compound.

KTP-NH2 had the highest effect on DISAGREGGATOR I P301L, reducing the level of stress down to almost 40% of the maximum level at a concentration of 100 µM.

In comparison, the ER stress that was chemically induced in DISAGGREGATOR I cells (with 1 µg/ml tunicamycin) was barely reduced to 80% with 100 µM compound, suggesting the specificity of action on the TAU P301L protein.

### In vivo Toxicology

The toxicological studies were carried out with rats (Wister, male) injected i.p. once a day with 3,23mg-100g body mass during seven days.

### Hepatotoxicity markers in the plasma.

Liver is the major organ of toxicity. Blood samples were collected and the following hepatotoxicity markers were assayed in the plasma:
- Alanine transaminase (ALT),
- Aspartate transaminase (AST)
- Alkaline phosphatase (ALP)
- Total bilirubin (TBIL)
- Gamma glutamyl transpeptidase (GGT)

Increased levels of the liver enzymes ALT, AST, ALP and GGT in the plasma indicate lesions in liver. Increased RBL would be a sign of faulty bilirubin production/hemolysis and/or bilirubin metabolism (in liver).

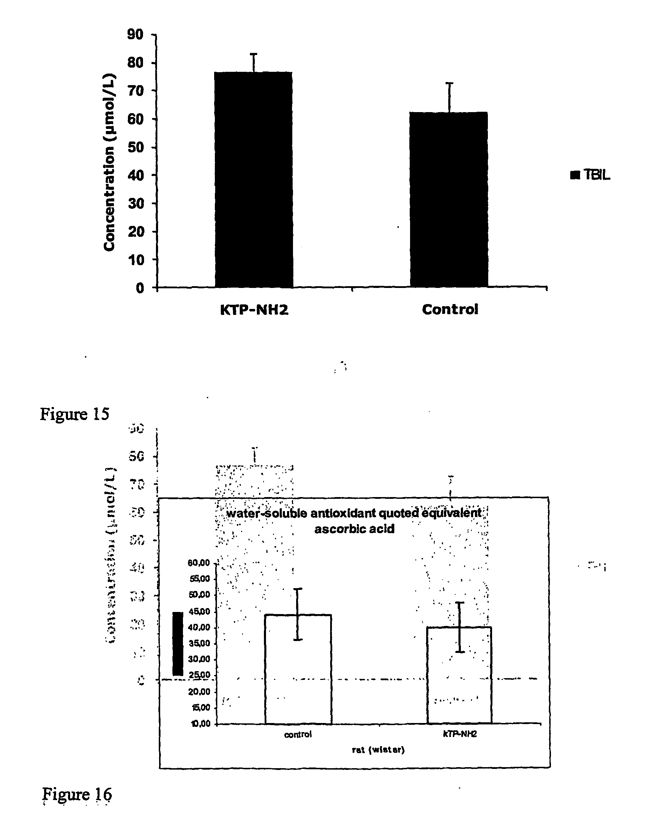

The results for levels of AST, ALT and ALP are shown in Figure 14. The results for the level of TBILI are shown in Figure 15.

Control and KTP-NH₂-treated animals show identical results: no toxic effects were detected (see figures 14 and 15).

Furthermore, in the test animals GGT was only present in trace amounts, below the detection limit of the analytical spectrophotometers.

### Determination of antioxidant capacity in blood samples

Metabolization of KTP-NH2 in the organism might lead to an increase in its metabolite products and to the subsequent production of reactive oxygen species (ROS). As increased ROS levels are potentially harmful.

KTP-NH₂ potential to induce changes in antioxidant capacities in the plasma of animals was explored. Total antioxidant capacity of lipid-soluble (ACL) and water-soluble (ACW) constituents were determined.

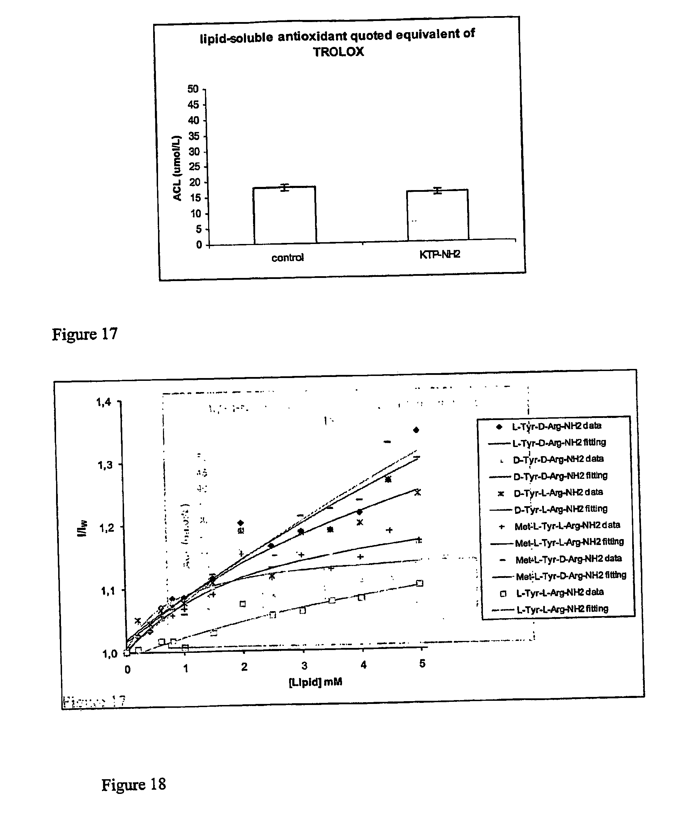

Accordingly with Figures 16 and 17, KTP-NH₂ does not induce significant modifications of basal antioxidant capacity in plasma of treated animals.

### Detection of histological alterations

For investigation of histological alterations in liver, kidney and spleen hematoxylin/eosin-stained sections of fixed tissue were used. Hematoxylin/eosin-stained sections were examined by an experienced pathologist.

There were no signs of hepatic necrosis in rat's liver and no histological modifications were found in kidney and spleen of these animals.

### In vitro ADMET Absorption, Distribution, Metabolism, Excretion and Toxicology.

ADMET deficient properties are one of the major factors that cause failures during drug development. Therefore, the ADME characteristics of KTP-NH2 were evaluated *in vitro* and the compound proved to have attributes of a good drug candidate.

### Metabolic stability

Many compounds can never become a drug because they are rapidly metabolized in the liver. It is important to confirm that metabolic stability is adequate to the desired distribution of compound throughout the body. The *in vitro* metabolic stability of KTP-NH2 was tested using a microsomal preparation from human liver, which contains all the cytochrome P450 (CYP) isozymes and other metabolizing enzymes (Kuhnz W. and Gieschen H. 1998. Predicting the oral bioavailability of 19-nortestosterone progestins in vivo from their metabolic stability in human liver microsomal preparations in vitro. Drug Metab. Dispos. 26: 1120-1127). Results obtained showed that KTP-NH2 is metabolically stable, as 93% of the compound remained after 1-hour incubation with the microsomal preparation.

### CYP inhibition

If a compound inhibits cytochrome P450 (CYP) isozymes this will lead to the accumulation of endogenous substances or other drugs that are substrates of the inhibited CYP, leading to potential toxicity. CYP3A4 is one of the most important enzymes involved in the metabolism of xenobiotics in the body, promoting the oxidation of the largest range of substrates of all the CYPs and is present in the largest quantity of all the CYPs in the liver. KTP-NH2 was shown not to inhibit CYP3A4 in a specific *in vitro* inhibition assay (Dierks E.A., Stams K.R:, Lim H.K., Cornelius G., Zhang H. and Ball S.E. 2001. A method for the simultaneous evaluation of the activities of seven major human drug-metabolizing cytochrome P450s using an in vitro cocktail of probe substrates and fast gradient liquid chromatography tandem mass spectrometry. Drug Metab. Dispos. 29: 23-29) using two traditional CYP3A4 probe substrates, midazolan and testosterone.

### Cytotoxicity

Cytotoxicity was assessed with a cell-based assay using human hepatocytes. Cell death was assayed by quantifying plasma membrane damage or rupture through measurement of the release of lactate dehydrogenase (LDH) (Legrand, C. et al. 1992. Lactate dehydrogenase (LDH) activity of the cultured eukaryotic cells as marker of the number of dead cells in the medium. J. Biotechnol. 25: 231-243), a stable cytoplasmic enzyme present in most cells. Determination of cell viability was performed by quantifying ATP (Cree I.A and Andreotti P.E. 1997. Measurement of cytotoxicity by ATP- based luminescence assay in primary cell cultures and cell lines. Toxicology in vitro, 11: 553-556), a marker for cell viability because it is present in all metabolically active cells and the concentration declines very rapidly when the cells undergo necrosis or apoptosis. Treatment of human hepatocytes with increasing concentrations of KTP-NH2 and subsequent LDH and ATP determination revealed that the compound is low hazardous (LC50>1251µM).

### Plasma stability

Drugs are exposed in plasma to enzymatic processes (proteinases, esterases), they can undergo intramolecular rearrangement or bind irreversibly (covalently) to proteins. Compounds which are not stable in plasma have inherent liability as drug candidates, as they are less capable to reach a sufficient concentration at their site of pharmacological activity..KTP-NH2 shows 14-21% stability in human plasma after 1h incubation (Singh R., Chang S.Y. and Talor L.C. 1996. In vitro metabolism of a potent HIV-protease inhibitor (141W94) using rat, monkey and human liver S9. Rapid Commun. Mass Spectrom. 10: 1019-1026), being metabolized into its constituent amino acids, arginine and tyrosine.

### Improved KTP-NH2 analogues

In order to increase the potential of KTP-NH2 as a drug for systemic administration, derivatives with improved plasma stability were generated, and tested, including different isomers of KTP-NH2 and methylated versions of KTP-NH2 isomers:
- L-Tyrosyl-D-Arginine-NH2
- D-Tyrosyl-D-Arginine-NH2
- D-Tyrosyl-L-Arginine-NH2
- Methyl-L-Tyrosyl-L-Arginine-NH2
- Methyl-L-Tyrosyl-D-Arginine-NH2

These analogues were tested for plasma serum stability, in comparison with KTP-NH2. All of these KTP-NH2 analogues revealed reduced degradation and displayed high stability values, ranging from 79% to 99%, after 1-hour incubation, see table below.

| **Compound** | **Test concentration [uM]** | **Condition** | **Mean Parent Remaining (%)** |
|---|---|---|---|
| L-Tyr-L-Arg-NH2 | 400 | Human Serum | 14,00 |
| L-Tyr-D-Arg-NH2 | 400 | Human Serum | 78,78 |
| D-Tyr-O-Arg-NH2 | 400 | Human Serum | 98,37 |
| D-Tyr-L-Arg-NH2 | 400 | Human Serum | 98,01 |
| Me-L-Tyr-L-Arg-NH2 | 400 | Human Serum | 88,98 |
| Me-L-Tyr-D-Arg-NH2 | 400 | Human Serum | 98,47 |

In order to confirm that these plasma-stable KTP-NH2 derivatives maintained their lipophilicity, their potential interaction with human cell membranes was assessed. Again, we performed biophysical studies using fluorescent methodologies (Santos N.C., Prieto M. and Castanho M.A. 2003. Quantifying molecular partition into model systems of biomembranes: an emphasis on optical spectroscopic methods. Biochim Biophys Acta 1612: 123-135.). A good interaction of all of the improved derivatives with the lipid bilayers was corroborated by the results shown in Figure 18, suggesting that they maintain the high lipophilic characteristics.

### Synthesis of Compounds of the Invention

### Synthesis of Tyr-Arg-NH₂

### Synthesis of Boc-Tyr(tBu)-Arg-NH₂ (3)

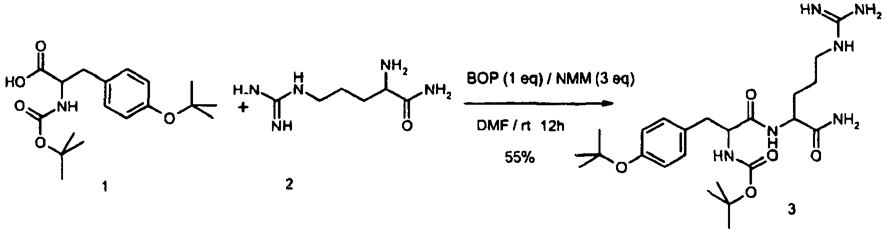

N-methylmorpholine (NMM) (3.3 mL, 30 mmol) was added to a solution of Boc-Tyr(tBu)-OH (**1**) (3.374 g, 10 mmol) in *N,N*-dimethylforamide (DMF) (40 mL), and the resulting mixture was stirred at room temperature for 1 h. Then, benzotriazole-1-yl-oxy-tris-(dimethylamino)-phosphoniumfluorophosphate (BOP) (4.42 g, 10 mmol) and H-Arg-NH₂ x 2HCl (**2**) (2.46 g, 10 mmol) were added. The resulting reaction mixture was stirred overnight at room temperature. Upon completion of the reaction (Thin layer chromatography (TLC) monitoring), the reaction mixture was filtered. The resulting solution was diluted with ethyl acetate (100 mL), washed with saturated sodium bicarbonate (3 x 50 mL), water (100 mL), 1M aqueous potassium hydrogenosulfate (3 x 50 mL), and brine (50 mL). The organic layer was dried over magnesium sulfate, filtered and concentrated *in vacuo* to afford compound **3** (2.7 g, 55% yield) as a colourless solid. The structure of compound 3 was confirmed by ¹H-NMR.

### Synthesis of Tyr-Arg-NH₂ (HCl) (4)

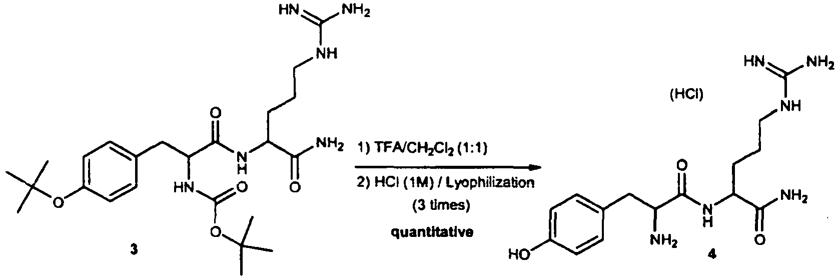

Compound **3** (2.6 g, 5.28 mmol) was dissolved in CH₂Cl₂ (8 mL) and the solution was cooled in an ice bath. Trifluoroacetic acid (TFA) (8 mL) was added dropwise and the resulting mixture was stirred at 0°C for 1-2 h. Upon completion of the reaction (TLC monitoring), the solvent was removed under reduced pressure. The resulting residue was triturated with ether, collected and dried *in vacuo.* The resulting white solid was dissolved in 1M aqueous HCl and lyophylized. This process was repeated three times to afford Tyr-Arg-NH₂ (**4**) (1.76 g, 100% yield) as a hydrochloride salt. The structure of compound **4** was confirmed by ¹H-NMR and High resolution mass spectrometry (HRMS) electrospray ionisation (ESI).

### Synthesis of Tyr-Arg-OH

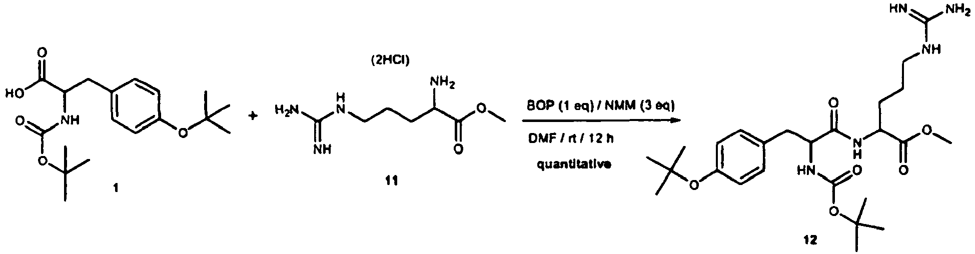

### Synthesis of Boc-Tyr(tBu)-Arg-OMe (12)

NMM (3.3 mL, 30 mmol) was added to a solution of Boc-Tyr(tBu)-OH (**1**) ( 3.374 g, 10 mmol) in DMF (40 mL) and the resulting mixture was stirred at room temperature for 1 h. Then, BOP (4.42 g, 10 mmol) and H-Arg-OMe x 2HCl (**11**) (2.88 g, 10 mmol) were added. The resulting reaction mixture was stirred overnight at room temperature. Upon completion of the reaction (TLC monitoring), the reaction mixture was filtered. The resulting solution was diluted with ethyl acetate (100 mL) washed with saturated sodium bicarbonate (3 x 50 mL), water (100 mL), 1M aqueous potassium hydrogenosulfate (3 x 50 mL), and brine (50 mL). The organic layer was dried over magnesium sulfate, filtered and concentrated *in vacuo* to leave compound **12** (5.07 g, 100%) as a colourless solid. The structure of compound **12** was confirmed by ¹H-NMR and HRMS(ESI).

### Synthesis of Boc-Tyr(tBu)-Arg-OH (13)

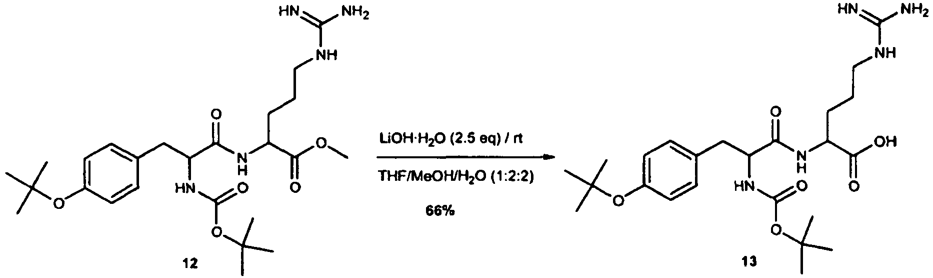

LiOH monohydrate (10.33 g, 24.62 mmol) was added to a solution of compound **12** (5.0 g, 9.85 mmol) in THF/MeOH/water (1:2:2, 82 mL), and the reaction mixture was stirred overnight at room temperature. Upon completation of the reaction (TLC monitoring), the organic solvents were removed under reduced pressure. The resultant aqueous solution was adjusted to pH 2 by addition of glacial acetic acid upon which the expected Boc-Tyr(tBu)-Arg-OH **13** was precipitated. The solid was collected by filtration, washed with cold water and dried *in vacuo* to afford compound **13** (3.21 g, 66% yield) as a colourless solid. The structure of compound **13** was confirmed by ¹H-NMR and HRMS(ESI).

### Synthesis of Tyr-Arg-OH (HCl) (14)

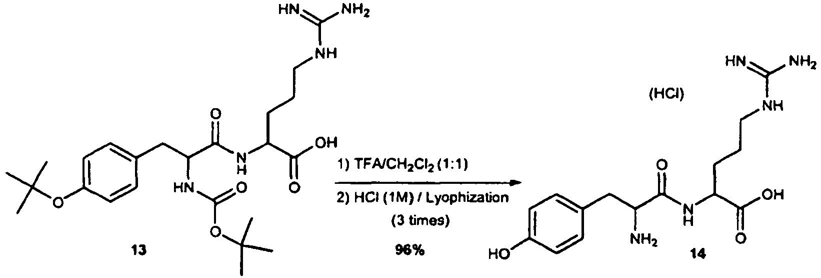

Compound **13** (3.10 g, 6.28 mmol) was dissolved in CH₂Cl₂ (9.5 mL) and the solution was cooled in an ice bath. TFA (9.5 mL) was added dropwise and the resulting mixture was stirred at 0°C for 1-2 h. Upon completion of the reaction (TLC monitoring), the solvent was removed under reduced pressure. The resulting residue was triturated with ether, collected and dried *in vacuo.* The resulting white solid was dissolved in 1M aqueous HCl and lyophylized. This process was repeated three times to afford Tyr-Arg-OH (**14**) (2.03 g, 96% yield) as a hydrochloride salt. The structure of compound **14** was confirmed
by ¹H-NMR and HRMS(ESI).

### Solid synthesis of KTP derivatives:

Prior to the first aminoacid coupling, both swelling and Fmoc deprotection of the resin (Rink amide HBMA resin) are required. To accomplish this, the resin was left for 20 min in dichloromethane (DCM, 2mL) and then 20 min in Dimethylformamide (DMF, 2mL). The solution was removed by vacuum filtration being the resin treated with a solution of piperidine in DMF (3:7, 2.5mL) for a total of 12 min. The resin was then filtrated again. For amino acid coupling, a solution in DMF (2.5 mL) of the Fmoc-protected aminoacid (3eq), N-Ethyldiisopropylamine (DIEA, 3eq) and O-Benzotriazole-N,N,N',N'-tetramethyl-uronium-hexafluoro-phosphate, (HBTU, 3eq) was added to the resin and left for 3 hours, with constant stirring. The Kaiser test was applied for evaluation of the coupling success. For the removal of the Fmoc group of the amino acid, piperidine in DMF was used as before. The second amino acid was coupled and deprotected using the conditions used for the first amino acid coupling, and the reaction was assessed by Kaiser test. To unlink the peptide from the resin, the reaction mixture was stirred for 2 hours in a solution of Trifluoroacetic acid (TFA):water:triisopropylsilane (TIS) (95:2.5:2.5, 2.5mL) affording the free peptide. Between each step the reaction crude was washed with DCM and DMF (6x/1min each). The solution was 3 times washed with Diethyl ether and centrifuged (5,000 rpm; 5 min). The precipitate obtained is the peptide. The precipitate was dissolved in water and lyophilized, obtaining each KTP derivative as a colourless solid (yield ranging between 60% and 80%). The purity of each peptide was analysed by HPLC.

| | 1^{st} amino acid | 2^{nd} amino acid |
|---|---|---|
| L-Tyr-D-Arg-NH₂ | D-Fmoc-Arg(Pmc)-OH | L-Fmoc-Tyr(tBu)-OH |
| D-Tyr-D-Arg-NH₂ | D-Emoc-Arg(Pmc)-OH | D-Fmoc-Tyr(tBu)-OH |
| D-Tyr-L-Arg-NH₂ | L-Fmoc-Arg(Pmc)-OH | D-Fmoc-Tyr(tBu)-OH |
| Me-L-Tyr-L-Arg-NH₂ | L-Fmoc-Arg(Pmc)-OH | L-Fmoc-Me-Tyr(tBu)-OH |
| Me-L-Tyr-L-Arg-NH₂ | D-Fmoc-Arg(Pmc)-OH | L-Fmoc-Me-Tyr(tBu)-OH |

### Abbreviations

- BOP: benzotriazole-1-yl-oxy-tris-(dimethylamino)-phosphoniumfluorophosphate
- DMF: N,N-dimethylforamide
- dr: diastereoisomeric ratio
- ESI: electrospray ionisation
- WMM: N-methylmorpholine
- ¹H-NMR: Proton Nuclear Magnetic Resonance
- HPLC: High Perforance Liquid Chromatography
- HRMS: High Resolution Mass Spectrometry
- rt: room temperature
- TFA: trifluoroacetic acid
- TLC: Thin Layer Chromatography
- HBMA: Hydroxy butyl methacrylate

The inventors created a yeast-based screening application for the detection of compounds that act as modulators of the toxicity (neurodegeneration) for neurons (not pain) induced by the expression of tau P301L, a pathological isoform of tau protein. This yeast-based application is designated as DISAGGREGATOR I P301L and provides a valuable tool for the high throughput screening of tau modulators. It is a model-based system where the cellular environment produced upon P301L expression is recapitulated and cell toxicity is measured and quantified. Compounds that have the potential to modulate pathological pathways activated by the expression of P301L are detected through a reduction in the cytotoxic readout. Tau protein is expressed in central nerve cells and, consequently, tauopathies are diseases of the central nervous system. This implies that a therapeutic compound for tauopathies must be able to access the central nervous system by penetrating the blood-brain barrier. We tested KTP-NH2 in the DISAGGREGATOR P301L system. Results were very impressive for KTP-NH2 to be used as a drug for tau-associated pathologies. Therefore, after obtaining the proof of concept confirmation-of KTP-NH2 potential as a tau modulator, we developed a series of derivatives with a higher plasma-stability. The present invention protects the use of KTP-NH2 and its derivatives according to the claims in the treatment of tau-induced cytotoxicities.

## Claims

1. A compound of formula (I) 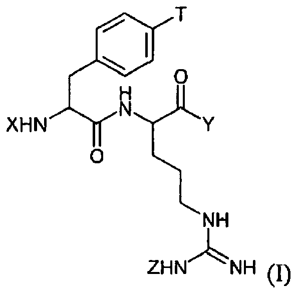
wherein X is hydrogen, R¹, R¹C(O) or R¹CO₂,
wherein R¹ is C₁₋₂₀ alkyl, aryl, arylalkyl, alkyloxy or arylalkyloxy,
wherein Y is OR², NHR³ or N(R³)₂,
wherein R² is C₁₋₂₀ alkyl and each R³ is independently hydrogen or a C₁₋₄ alkyl;
wherein T is OR⁴, NHR⁵ or N(R⁵)₂,
wherein R⁴ is hydrogen or C₁₋₂₀ alkyl and each R⁵ is independently hydrogen or a C₁₋₄ alkyl;
wherein Z is hydrogen, R⁶, R⁶C(O) or R⁶CO₂,
wherein R⁶ is C₁₋₂₀ alkyl, aryl, arylalkyl, alkyloxy or arylalkyloxy,
for use in the prevention and/or treatment of a tauopathy.

2. The compound for use as claimed in claim 1 wherein X is hydrogen, Y is NH₂, T is hydroxyl and Z is hydrogen.

3. The compound for use as claimed in claim 1 or claim 2 wherein the tauopathy is Corticobasal Degeneration (CBD), Frontotemporal Dementia with Parkinsonism linked to Chromosome 17 (FTDP17), Pick's Disease (PiD), Progressive Supranuclear Palsy (PSP), Alzheimer's Disease (AD), Multiple System Atrophy (MSA), Parkinson's Disease (PD), Dementia with Lewy Bodies (DLB), Down's Syndrome or Parkinsonism-Dementia Complex of Guam.

4. The use of the compound of formula (I) as defined in claim 1 or claim 2 in the manufacture of a medicament for the prevention and/or treatment of a tauopathy.

5. The use as claimed in claim 4 for the prevention and/or treatment of Corticobasal Degeneration (CBD), Frontotemporal Dementia with Parkinsonism linked to Chromosome 17 (FTDP17), Pick's Disease (PiD), Progressive Supranuclear Palsy (PSP), Alzheimer's Disease (AD), Multiple System Atrophy (MSA), Parkinson's Disease (PD), Dementia with Lewy Bodies (DLB), Down's Syndrome or Parkinsonism-Dementia Complex of Guam.

6. A pharmaceutical composition comprising the compound of formula (I) as defined in claim 1 or claim 2 and a pharmaceutically acceptable excipient for use in the prevention and/or treatment of a tauopathy.

7. A pharmaceutical composition as claimed in claim 6 for use in the prevention and/or treatment of Corticobasal Degeneration (CBD), Frontotemporal Dementia with Parkinsonism linked to Chromosome 17 (FTDP17), Pick's Disease (PiD), Progressive Supranuclear Palsy (PSP), Alzheimer's Disease (AD), Multiple System Atrophy (MSA), Parkinson's Disease (PD), Dementia with Lewy Bodies (DLB), Down's Syndrome or Parkinsonism-Dementia Complex of Guam.

## Patentansprüche

1. Verbindung mit der Formel (I) 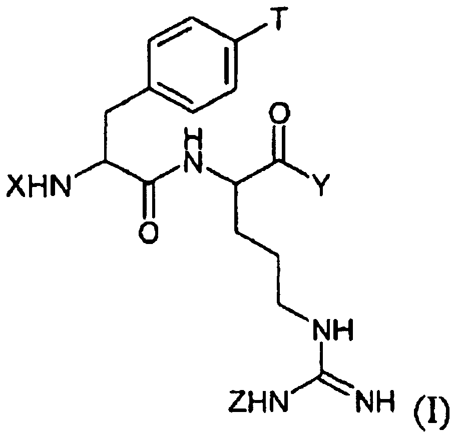
wobei X für Wasserstoff, R¹, R¹C(O) oder R¹CO₂ steht,
wobei R¹ für C₁₋₂₀-Alkyl, Aryl, Arylalkyl, Alkyloxy oder Arylalkyloxy steht,
wobei Y für OR², NHR³ oder N(R³)₂ steht,
wobei R² für C₁₋₂₀-Alkyl steht und jedes R³ unabhängig für Wasserstoff oder ein C₁₋₄-Alkyl steht;
wobei T für OR⁴, NHR⁵ oder N(R⁵)₂ steht,
wobei R⁴ für Wasserstoff oder C₁₋₂₀-Alkyl steht und jedes R⁵ unabhängig für Wasserstoff oder ein C₁₋₄-Alkyl steht;
wobei Z für Wasserstoff, R⁶, R⁶C(O) oder R⁶CO₂ steht,
wobei R⁶ für C₁₋₂₀-Alkyl, Aryl, Arylalkyl, Alkyloxy oder Arylalkyloxy steht,
zur Verwendung bei der Vorbeugung und/oder Behandlung einer Tauopathie.

2. Verbindung zur Verwendung nach Anspruch 1, wobei X für Wasserstoff steht, Y für NH₂ steht, T für Hydroxyl steht und Z für Wasserstoff steht.

3. Verbindung zur verwendung nach Anspruch 1 oder Anspruch 2, wobei die Tauopathie kortikobasale Degeneration (CBD), frontotemporale Demenz mit Parkinsonismus verknüpft mit Chromosom 17 (FTDP17), Pick-Krankheit (PiD), progressive supranukleäre Paralyse (PSP), Alzheimer-Krankheit (AD), Multisystematrophie (MSA), Parkinson-Krankheit (PD), Lewy-Körperchen-Demenz (DLB), Down-Syndrom oder Guam-Parkinson-Demenz-Komplex ist.

4. Verwendung der Verbindung mit der Formel (I) wie in Anspruch 1 oder Anspruch 2 definiert, bei der Herstellung eines Medikaments zur Vorbeugung und/oder Behandlung einer Tauopathie.

5. Verwendung nach Anspruch 4 zur Vorbeugung und/oder Behandlung von kortikobasaler Degeneration (CBD), frontotemporaler Demenz mit Parkinsonismus verknüpft mit Chromosom 17 (FTDP17), Pick-Krankheit (PiD), progressiver supranukleärer Paralyse (PSP), Alzheimer-Krankheit (AD), Multisystematrophie (MSA), Parkinson-Krankheit (PD), Lewy-Körperchen-Demenz (DLB), Down-Syndrom oder Guam-Parkinson-Demenz-Komplex.

6. Pharmazeutische Zusammensetzung, die die Verbindung mit der Formel (I), wie in Anspruch 1 oder Anspruch 2 definiert, und einen pharmazeutisch verträglichen Exzipienten umfasst, zur Verwendung bei der Vorbeugung und/oder Behandlung einer Tauopathie.

7. Pharmazeutische Zusammensetzung nach Anspruch 6 zur Verwendung bei der Vorbeugung und/oder Behandlung von kortikobasaler Degeneration (CBD), frontotemporaler Demenz mit Parkinsonismus verknüpft mit Chromosom 17 (FTDP17), Pick-Krankheit (PiD), progressiver supranukleärer Paralyse (PSP), Alzheimer-Krankheit (AD), Multisystematrophie (MSA), Parkinson-Krankheit (PD), Lewy-Körperchen-Demenz (DLB), Down-Syndrom oder Guam-Parkinson-Demenz-Komplex.

## Revendications

1. Composé de formule (I) : 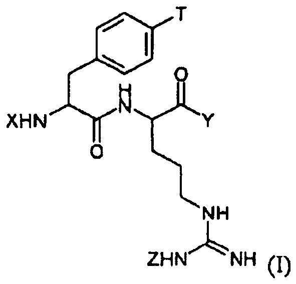
dans laquelle X est un atome d'hydrogène, R¹, R¹C (O) ou R¹CO₂,
dans laquelle R¹ est un groupe alkyle en C_{1 à 20}, aryle, arylalkyle, alkyloxy ou arylalkyloxy,
dans laquelle Y est OR², NHR³ ou N(R³)₂,
dans laquelle R² est un groupe alkyle en C_{1 à 20} et chaque R³ est indépendamment un atome d'hydrogène ou un groupe alkyle en C_{1 à 4} ;
dans laquelle T est OR⁴, NHR⁵ ou N(R⁵)₂,
dans laquelle R⁴ est un atome d'hydrogène ou un groupe alkyle en C_{1 à 20} et chaque R⁵ est indépendamment un atome d'hydrogène ou un groupe alkyle en C_{1 à 4} ;
dans laquelle Z est un atome d'hydrogène, R⁶, R⁶C (O) ou R⁶CO₂,
dans laquelle R⁶ est un groupe alkyle en C_{1 à 20}, aryle, arylalkyle, alkyloxy ou arylalkyloxy,
à utiliser dans la prévention et/ou le traitement d'une tauopathie.

2. Composé à utiliser selon la revendication 1, dans lequel X est un atome d'hydrogène, Y est un NH₂, T est un groupe hydroxyle et Z est un atome d'hydrogène.

3. Composé à utiliser selon la revendication 1 ou la revendication 2, dans lequel la tauopathie est la dégénérescence corticobasale (DCB), la démence frontotemporale avec parkinsonisme liée au chromosome 17 (DFTP17), la maladie de Pick (MPi), la paralysie supranucléaire progressive (PSP), la maladie d'Alzheimer (MA), l'atrophie multisystématisée (AMS), la maladie de Parkinson (MP), la démence à corps de Lewy (DCL), le syndrome de Down ou le syndrome de Guam.

4. Utilisation du composé de formule (I) tel que défini dans la revendication 1 ou la revendication 2, dans la fabrication d'un médicament pour la prévention et/ou le traitement d'une tauopathie.

5. Utilisation selon la revendication 4, pour la prévention et/ou le traitement de la dégénérescence corticobasale (DCB), la démence frontotemporale avec parkinsonisme liée au chromosome 17 (DFTP17), la maladie de Pick (MPi), la paralysie supranucléaire progressive (PSP), la maladie d'Alzheimer (MA), l'atrophie multisystématisée (AMS), la maladie de Parkinson (MP), la démence à corps de Lewy (DCL), le syndrome de Down ou le syndrome de Guam.

6. Composition pharmaceutique comprenant le composé de formule (I) tel que défini dans la revendication 1 ou la revendication 2 et un excipient pharmaceutiquement acceptable à utiliser dans la prévention et/ou le traitement d'une tauopathie.

7. Composition pharmaceutique selon la revendication 6, à utiliser dans la prévention et/ou le traitement de la dégénérescence corticobasale (DCB), la démence frontotemporale avec parkinsonisme liée au chromosome 17 (DFTP17), la maladie de Pick (MPi), la paralysie supranucléaire progressive (PSP), la maladie d'Alzheimer (MA), l'atrophie multisystématisée (AMS), la maladie de Parkinson (MP), la démence à corps de Lewy (DCL), le syndrome de Down ou le syndrome de Guam.
